# EUROPEAN PATENT SPECIFICATION

(11) **EP 2 211 825 B1**
(45) Date of publication and mention of the grant of the patent: **20.08.2014**
(21) Application number: 08850444.4
(22) Date of filing: 12.11.2008
(51) Int. Cl.: C08F 8/44, A61Q 17/04, A61Q 13/00, A61K 8/11, A61K 8/81

(54) **ENCAPSULATED LOW VISCOSITY HYDROPHOBIC LIQUID ACTIVES**
VERKAPSELTE NIEDERVISKOSE HYDROPHOBE FLÜSSIGWIRKSTOFFE
INGRÉDIENTS ACTIFS LIQUIDES, HYDROPHOBES, À FAIBLE VISCOSITÉ ET ENCAPSULÉS

(30) Priority: 16.11.2007 US 988484 P
(43) Date of publication of application: 04.08.2010
(73) Proprietor: Dow Global Technologies LLC, Midland, MI 48674 (US)
(72) Inventor: MALOTKY, David, L., Midland MI 48640 (US); ZHANG, Xiaodong, Livingston NJ 07039 (US)
(74) Representative: Beck Greener
(86) International application number: PCT/US2008/083148
(87) International publication number: WO 2009/064739

(56) References cited:
- EP-A- 0 361 677
- EP-A- 0 410 099
- EP-A- 1 797 946
- WO-A-97/47288
- WO-A-03/082232
- GB-A- 1 096 946
- US-A- 4 766 012

## Description

### Field

The present invention relates to personal care compositions.

### Background

Many personal care compositions contain water insoluble actives. Such actives may exhibit a tendency to degrade in the formulation. For example, some vitamins are photolabile. While provitamins can be used in place of such a component, they are typically less efficacious. Moreover, certain components can have undesirable interactions if present together in a formulation. For example, aside from irritancy and aesthetic difficulties, sunscreen formulators have also previously been challenged by the fact that octinoxate and avobenzone are incompatible, in addition to each being photolabile.

In another example, fragrances can evaporate, thereby decreasing consumer satisfaction. In yet another example of the difficulties of incorporating hydrophobic actives into personal care compositions, certain flavors can destabilize oral care formulations.

One strategy to overcome such difficulties is to insulate or encapsulate the hydrophobic active. In the past, encapsulation has required high shear and/or solvent exchange processes. Thus, what is needed are improved methods and compositions containing encapsulated or otherwise protected hydrophobic personal care actives.

### Summary

The present invention provides a method for protecting low viscosity hydrophobic liquid personal care actives, comprising forming a mixture comprising an ethylene/acrylic acid copolymer capable of being surface active, water, and a base, and combining the active with the mixture to form suspended active particles, and precipitating the copolymer through addition of an acid to lower the pH of the mixture, thereby forming shells around the active particles.

In one embodiment, the present invention provides methods for encapsulating low viscosity hydrophobic liquid personal care actives with an ethylene/acrylic acid copolymer, comprising raising the pH of an ethylene/acrylic acid copolymer mixture above about 7, dispersing the active in the mixture, and precipitating the copolymer, thereby forming a shell around the active.

### Detailed Dercription

The present invention provides a method for protecting low viscosity hydrophobic liquid personal care actives, comprising forming a mixture comprising an ethylene/acrylic acid copolymer capable of being surface active, water, and a base, and combining the active with the mixture to form suspended active particles, and precipitating the copolymer through addition of an acid to lower the pH of the mixture, thereby forming shells around the active particles.

The term "low viscosity" refers to less than 100,000 cps (100,000 mPa·s.), preferably less than 50,000 cps (50,000 mPa·s), and more preferably, less than 20,000 cps (20,000 mPa·s).

"Hydrophobic liquid," for purposes of this disclosure, refers to a liquid component that is more soluble in dodecane than in water. Such components generally have a log octanol/water partition coefficient greater than 1. Examples may be found in the CRC Handbook of Chemistry & Physics, edited by D. R. Linde, CRC Press, Florida, 74th Ed. (1993-94), Sec. 16, page 24 et seq*.*

"Personal care" relates to compositions to be topically applied to a person (including mouth, ear, and nasal cavities, but not ingested). Examples of personal care compositions include skin care products (e.g., facial cream, moisturizers, leave on and rinse off lotions, sunscreens, foundation, mascara, eye-liner and lipstick), oral care products (such as toothpastes and rinses), nail care products (such as polish and conditioners), and hair care products (including leave on and rinse off conditioners, styling gels and hairsprays). Similarly, for purposes of this specification, "personal care active" refers to any component that imparts a primary personal care benefit to a user, as opposed to solely facilitating creation of the formulation itself. Thus, for example, water is not an active. Examples of personal care actives include typical actives for skin care products (e.g., facial cream, moisturizers, leave on and rinse off lotions, sunscreens, foundation, mascara, eye-liner and lipstick), oral care products (such as toothpastes and rinses), nail care products (such as polish and conditioners), and hair care products (including leave on and rinse off conditioners, styling gels and hairsprays).

Examples of actives include antibacterial compounds (e.g. triclosan) in toothpaste, polypheols, flavinoids and isoflvinoids, coenzyme Q10 and derivatives thereof, carotene and derivatives thereof, salicylic acid and derivatives thereof, dehydroepiandrosterone (DHEA), hydrophobic polysaccharides, proteins, including enzymes and peptides, oil based pigment dispersions, and botanicals.

In one embodiment, the present invention provides that the personal care active is a vitamin, emollient, oil based pigment dispersions, sunscreen, essential oil, or fragrance. In one embodiment, the personal care active is octinoxate.

Examples of sunscreens include paraminobenzoic acid, avobenzone, cinoxate, dioxybenzone, homosalate, menthyl anthranilate, octocrylene, octyl methoxycinnamate, octyl salicylate, oxybenzone, padimate O, phenylbenzimidazole sulfonic acid, sulisobenzone, trolamine salicylate, titanium dioxide and zinc oxide, diethanolamine methoxycinnamate, digalloy trioleate, ethyl dihydroxypropyl PABA, glyceryl aminobenzoate, lawsone with dihydroxy acetone, and red petrolatum.

Examples of oil based pigment dispersions include pigment particles, including metal oxides, dispersed in a hydrophobic carrier liquid, such as, silicone oils, including, for example, polydimethylsiloxane, mineral oils, and alkyl benzoate. It is understood that this contemplates both coated and uncoated pigment particles.

Vitamins include Vitamin A and esters thereof, Vitamin D and derivatives thereof, Vitamins B3 and B5 and derivatives thereof, Vitamin E and esters thereof, Vitamin F and derivatives thereof, and Vitamin K.

Dyes include liposoluble dyes, such as Sudan red, DC Red 17, DC Green 6, β-carotene, soybean oil, Sudan brown, DC Yellow 11, DC Violet 2, DC Orange 5, and quinoline yellow.

Flavors include flavor oils, such as peppermint, wintergreen, citrus, fruit, vanilla, and cinnamon. Most flavors are hydrophobic, and thus contemplated.

Fragrances include any component which provides a pleasant scent. Examples include scents that are floral, ambery, woody, leather, chypre, fougère, musk, vanilla, fruit, and/or citrus. Fragrances are often oils obtained by extraction of natural substances or synthetically produced. In one embodiment, the fragrance is one of the essential oils.

In a preferred embodiment, the hydrophobic, cosmetically acceptable, personal care active, vitamin, dye, flavor, or fragrance, is one that is susceptible to reaction or degradation in the personal care composition, including evaporation, photo-degradation, oxidation, or any other processes which leave such a component less potent or effective. "Cosmetically acceptable" refers to ingredients typically used in personal care compositions, and is intended to underscore that materials that are toxic, irritating, or have an unpleasant odor when present in the amounts typically found in personal care compositions are not contemplated as part of the present invention.

The polyolefin acid copolymer is one that is capable of being surface active. The polyolefin acid copolymer is one that can transition between a surface active state and a non-surface active state.

In the surface active state, the polyolefin acid copolymer has partitioned to the interface between the hydrophobic active and the water, forming a protective gel encapsulating the active. For example, ethylene/acrylic acid copolymer becomes surface active upon combination with water and a base, provided that the pH is greater than about 7.

If the pH is then reduced below about 7, the ethylene/acrylic acid copolymer loses its surface active property and precipitates. Advantageously, this precipitation results in encapsulation of the active component that was suspended when the copolymer was surface active, thus forming a "hard" shell.

In one embodiment, methods of the present invention include precipitating the copolymer, thereby forming shells around the active particles. In one embodiment, the particle defined by the shell has a particle size of less than 2 µm, preferably less than 1.8 µm, preferably less than 1.6 µm, preferably less than 1.4 µm, preferably less than 1.2 µm, and preferably about 1 µm. The particle size is taken as the volume average diameter by light scattering with a Coulter LS230. In one embodiment, the particle defined by the shell has a volume average particle size in a range from about 2 µm to about 1 µm. Preferably, volume average particle size is in a range from about 1.5 µm to about 1 µm.

The ethylene/acrylic acid copolymer has from 9 to 22 weight percent of acrylic acid units, preferably 18 to 22 weight percent of acrylic acid units, preferably from 19 to 21 percent of acrylic acid units, and most preferably 20 acrylic acid units. Examples of commercially available ethylene/acrylic acid copolymers include those sold under the tradenames PRIMACOR 5980i, PRIMACOR 5986, and PRIMACOR 5990i, all available from The Dow Chemical Company, and NUCREL 2806, available from E.I. du Pont de Nemours and Company, Inc. Ethylene-acrylic acid and ethylene-methacrylic acid copolymers, are described in U.S. Pat. Nos. 4,599,392, 4,988,781, and 5,938,437.

In one embodiment, a short chain alcohol is included in the dispersion of the PRIMACOR polymer to reduce the amount of undispersed material after the addition of the base, as described in U.S. Pat. No. 3,798,194.

In one embodiment, the ratio of ethylene/acrylic acid copolymer to active in the mixture is 1:1 to 1:20, preferably 1:5 to 1:15, most preferably 1:10.

In one embodiment, the upper limit concentration of ethylene/acrylic acid copolymer in water is ~30%. The resulting dispersion can be diluted down, preferably to a dispersion that is between 10% and 26% ethylene/acrylic acid copolymer as the starting point to which the hydrophobic active is added.

In one embodiment, the ethylene/acrylic acid copolymer is present in the non-aqueous ingredients from about 4.5 weight percent to about 50 weight percent, preferably about 6.3 weight percent to about 17 weight percent, most preferably about 9 weight percent.

In one embodiment, the dispersion that is between 10% and 26% ethylene/acrylic acid copolymer as the starting point to which the hydrophobic active is added.

The mixture is formed by combining ethylene/acrylic acid copolymer and a base, and then adding water. In one embodiment, the base is NaOH, KOH, or triethanolamine.

The active preferably is added to the mixture.

In one embodiment, the active and the mixture are emulsified. Emulsification can be performed using any conventional method.

In one embodiment, during combination, the pH remains greater than about 7. If necessary, the pH can be maintained at above about 7 to prevent precipitation of the ethylene/acrylic acid copolymer.

In one embodiment, precipitating includes lowering the pH of the mixture. In one embodiment, the pH is lowered with citric acid.

In one embodiment, the present invention further includes adding a stabilizer to the mixture. Preferred stabilizers include nonionic surfactants, preferably those with an HLB range of 1 - 20.

In another embodiment, the present invention provides methods for encapsulating low viscosity hydrophobic liquid personal care actives with an ethylene/acrylic acid copolymer, comprising raising the pH of an ethylene/acrylic acid copolymer mixture above about 7, dispersing the active in the mixture, and precipitating the copolymer, thereby forming a shell around the active.

As shown in the attached examples, even in compositions where the polyolefin acid copolymer is not precipitated, i.e., the gel network, the active is still protected.

Other optional ingredients for personal care compositions of the present invention include cosmetically acceptable emollients, sunscreens, surfactants, emulsifiers, preservatives, rheology modifiers, colorants, preservatives, pH adjustors, propellants, reducing agents, fragrances, foaming agents, tanning agents, depilatory agents, flavors, astringents, antiseptics, deodorants, antiperspirants, insect repellants, bleaches, lighteners, anti-dandruff agents, adhesives, polishes, strengtheners, fillers, barrier materials, or biocides.

The moisturizers include 2-pyrrolidone-5-carboxylic acid and its salts and esters, alkyl glucose alkoxylates or their esters, fatty alcohols, fatty esters, glycols and, in particular, methyl glucose ethoxylates or propoxylates and their stearate esters, isopropyl myristate, lanolin or cetyl alcohols, aloe, silicones, propylene glycol, glycerol and sorbitol.

Conditioners include stearalkonium chloride, dicetyldimonium chloride, lauryl methyl gluceth-10 hydroxypropyldimonium chloride, and conditioning polymers such as polyquaternium-10, polyquaternium-24 and chitosan and derivatives thereof.

Examples of oils include hydrocarbon-based oils of animal origin, such as squalene, hydrocarbon-based oils of plant origin, such as liquid triglycerides of fatty acids comprising from 4 to 10 carbon atoms, for instance heptanoic or octanoic acid triglycerides, or alternatively, oils of plant origin, for example sunflower oil, corn oil, soybean oil, marrow oil, grapeseed oil, sesame seed oil, hazelnut oil, apricot oil, macadamia oil, arara oil, coriander oil, castor oil, avocado oil, jojoba oil, shea butter oil, or caprylic/capric acid triglycerides, MIGLYOL 810, 812 and 818 (from Dynamit Nobel), synthetic esters and ethers, especially of fatty acids, for instance the oils of formulae R¹COOR² and R¹OR² in which R¹ represents a fatty acid residue comprising from 8 to 29 carbon atoms and R² represents a branched or unbranched hydrocarbon-based chain comprising from 3 to 30 carbon atoms, for instance purcellin oil, isononyl isononanoate, isopropyl myristate, 2-ethylhexyl palmitate, 2-octyldodecyl stearate, 2-octyldodecyl erucate or isostearyl isostearate, hydroxylated esters, for instance isostearyl lactate, octyl hydroxystearate, octyldodecyl hydroxystearate, diisostearyl malate, triisocetyl citrate and fatty alcohol heptanoates, octanoates and decanoates, polyol esters, for instance propylene glycol dioctanoate, neopentyl glycol diheptanoate and diethylene glycol diisononanoate, pentaerythritol esters, for instance pentaerythrityl tetraisostearate, lipophilic derivatives of amino acids, such as isopropyl lauroyl sarcosinate, such as is sold under the name ELDEW SL 205 (from Ajinomoto), linear or branched hydrocarbons of mineral or synthetic origin, such as mineral oils (mixtures of petroleum-derived hydrocarbon-based oils), volatile or non-volatile liquid paraffins, and derivatives thereof, petroleum jelly, polydecenes, isohexadecane, isododecane, hydrogenated isoparaffin (or polyisobutene), silicone oils, for instance volatile or non-volatile polymethylsiloxanes (PDMS) comprising a linear or cyclic silicone chain, which are liquid or pasty at room temperature, especially cyclopolydimethylsiloxanes (cyclomethicones) such as cyclopentasiloxane and cyclohexadimethylsiloxane, polydimethylsiloxanes comprising alkyl, alkoxy or phenyl groups, which are pendent or at the end of a silicone chain, these groups comprising from 2 to 24 carbon atoms, phenyl silicones, for instance phenyl trimethicones, phenyl dimethicones, phenyltrimethylsiloxydiphenylsiloxanes, diphenyl dimethicones, diphenylmethyldiphenyltrisiloxanes 2-phenylethyltrimethyl siloxysilicates and polymethylphenylsiloxanes, fluoro oils such as partially hydrocarbon-based and/or partially silicone-based fluoro oils, ethers such as dicaprylyl ether (CTFA name: dicaprylyl ether), and C₁₂-C₁₅ fatty alcohol benzoates (FINSOLV TN from Finetex), mixtures thereof.

Oils include mineral oil, lanolin oil, coconut oil and derivatives thereof, cocoa butter, olive oil, almond oil, macadamia nut oil, aloe extracts such as aloe vera lipoquinone, jojoba oils, safflower oil, corn oil, liquid lanolin, cottonseed oil, peanut oil, hydrogenated vegetable oil, squalane, castor oil, polybutene, sweet almond oil, avocado oil, calophyllum oil, ricin oil, vitamin E acetate, olive oil, silicone oils such as dimethylopolysiloxane and cyclomethicone, linolenic alcohol, oleyl alcohol, and the oil of cereal germs.

Other suitable emollients include dicaprylyl ether, C₁₂₋₁₅ alkyl benzoate, DC 200 FLUID 350 silicone fluid (from Dow Corning Corp.), isopropyl palmitate, octyl palmitate, isopropyl myristate, hexadecyl stearate, butyl stearate, decyl oleate, acetyl glycerides, the octanoates and benzoates of C₁₂₋₁₅ alcohols, the octanoates and decanoates of alcohols and polyalcohols such as those of glycol and glyceryl, ricinoleates esters such as isopropyl adipate, hexyl laurate and octyl dodecanoate, dicaprylyl maleate, phenyltrimethicone, and aloe vera extract. Solid or semi-solid cosmetic emollients include glyceryl dilaurate, hydrogenated lanolin, hydroxylated lanolin, acetylated lanolin, petrolatum, isopropyl lanolate, butyl myristate, cetyl myristate, myristyl myristate, myristyl lactate, cetyl alcohol, isostearyl alcohol and isocetyl lanolate.

In some embodiments, the personal care composition further comprises an optional rheology modifier as a thickener. Examples of thickeners include polymers, for example, modified or unmodified carboxyvinyl polymers, such as the products sold under the names CARBOPOL and PEMULEN (INCI name: Acrylates/C₁₀₋₃₀ alkyl acrylate crosspolymer; available from Noveon), polyacrylates and polymethacrylates, such as the products sold under the names LUBRAJEL and NORGEL (from Guardian) or HISPAGEL (from Hispano Chimica), polyacrylamides, 2-acrylamido-2-methylpropanesulfonic acid polymers and polymers, which are optionally crosslinked and/or neutralized, for instance the poly(2-acrylamido-2-methylpropane-sulfonic acid) sold by Clariant (INCI name: ammonium polyacryldimethyltauramide), emulsified crosslinked anionic polymers of acrylamide and AMPS, such as those sold under the name SEPIGEL 305 (INCI name: Polyacrylamide/C13-14 Isoparaffin/Laureth-7; from Seppic) and under the name SIMULGEL 600 (INCI name: Acrylamide/Sodium acryloyldimethyltaurate polymer/Isohexadecane/Polysorbate 80; from Seppic), polysaccharide biopolymers, for instance xanthan gum, guar gum, carob gum, acacia gum, scleroglucans, chitin and chitosan derivatives, carrageenans, gellans, alginates, celluloses such as microcrystalline cellulose, carboxymethylcellulose, hydroxymethylcellulose and hydroxypropylcellulose, associative polymers, for instance associative polyurethanes, polymers comprising at least two hydrocarbon-based lipophilic chains comprising from 6 to 30 carbon atoms, separated with a hydrophilic sequence, such as the polyurethanes sold under the names SERAD FX1010, SERAD FX1100 and SERAD FX1035 (from Hüls America), RHEOLATE 255, RHEOLATE 278 and RHEOLATE 244 (INCI name: Polyether-urea-polyurethane; from Rheox), DW 1206F, DW 1206J, DW 1206B, DW 1206G, and ACRYSOL RM 2020 (from Röhm & Haas).

Colorants include pigments, which are used especially in make-up, including metal oxide pigments, titanium dioxide, optionally surface-treated, zirconium oxide or cerium oxide, zinc oxide, iron oxide (black, yellow or red), chromium oxide, manganese violet, ultramarine blue, chromium hydrate and ferric blue, carbon black, pigments of barium, strontium, calcium or aluminum (for example D&C or FD&C), cochineal carmine, mica coated with titanium or with bismuth oxychloride, titanium mica with iron oxides, titanium mica with, especially, ferric blue or chromium oxide, titanium mica with an organic pigment, nacreous pigments based on bismuth oxychloride, goniochromatic pigments, for example pigments with a multilayer interference structure, reflective pigments, for example particles with a silver-coated glass substrate, glass substrate coated with nickel/chromium/molybdenum alloy, glass substrate coated with brown iron oxide, particles comprising a stack of at least two polymer layers, for instance MIRROR GLITTER (from 3M).

Dyes include water-soluble dyes such as copper sulfate, iron sulfate, water-soluble sulfopolyesters, rhodamines, natural dyes, for instance carotene and beetroot juice, methylene blue, caramel, the disodium salt of tartrazine and the disodium salt of fuschin, and mixtures thereof. Liposoluble dyes from the list above may also optionally be used.

Preservatives include alcohols, aldehydes, methylchloroisothiazolinone and methylisothiazolinone, p-hydroxybenzoates, and in particular methylparaben, propylparaben, glutaraldehyde and ethyl alcohol.

The pH adjustors, include inorganic and organic acids and bases and in particular aqueous ammonia, citric acid, phosphoric acid, acetic acid, and sodium hydroxide.

Reducing agents include ammonium thioglycolate, hydroquinone and sodium thioglycolate.

Fragrances may be aldehydes, ketones, or oils obtained by extraction of natural substances or synthetically produced as described above. Often, fragrances are accompanied by auxiliary materials, such as fixatives, extenders, stabilizers and solvents.

Biocides include antimicrobials, bactericides, fungicides, algaecides, mildicides, disinfectants, antiseptics, and insecticides.

The amount of optional ingredients effective for achieving the desired property provided by such ingredients can be readily determined by one skilled in the art.

### Examples

The following examples are for illustrative purposes only and are not intended to limit the scope of the present invention. All percentages are by weight unless otherwise specified.

### Example 1

Exemplary personal care compositions contain the components recited in TABLE 1. **TABLE 1.**

| | **Batch 1** | **Batch 2 (Comparative)** |
|---|---|---|
| White Tea Mod 4 fragrance (Fragrance Resources) | 1% | 1% |
| CARBOPOL ULTREZ 10 (Noveon) | 0.2% | 0.2% |
| PRIMACOR 5990i dispersion (25.3% solids) | 10% | -- |

The components are combined using a standard overhead mixer at a mixing speed of about 250 rpm for about 15 minutes.

### Example 2

Formulations made substantially according to the protocol described above in Example 1 were made, and tested for fragrance intensity via a fragrance panel study. 0.15 g of each batch were applied to the forearms of each of 5 panelists, and the fragrance intensity was compared after about 30 minutes. Four out of five panelists selected Batch 1 as having higher fragrance intensity.

### Example 3

Exemplary personal care compositions contain the components recited in TABLE 2.

**TABLE 2**

| | **Batch 3 (Inventive Gel)** | **Batch 4 (Inventive Precipitate)** | **Batch 5 (Comparative)** |
|---|---|---|---|
| Octinoxate | 33.6% | 19.53% | 64.6% |
| PRIMACOR 5990i dispersion (25.3% solids) | 66.4% | 77.17% | -- |
| LAURETH-4 | -- | -- | 2.1% |
| LAURETH-23 | -- | -- | 2.1% |
| POLOXAMER 331 | -- | 0.5% | -- |
| Citric Acid | -- | 2.8% | -- |

Batches 3 and 4 are different forms of the present invention, the former being an active encapsulated in a gel network, the latter being the result of adding acid to the gel, thereby precipitating a hard shell encapsulated active. The Batch 3 and Batch 5 formulation in TABLE 2 were combined by homogenization with an IKA ULTRA TURRAX mixer at 19,000 rpm for 1 minute. The first two ingredients of Batch 4 were also combined in this fashion, after which the mixture was moved over to a propeller mixer at 250 rpm and the POLOXAMER 331 and citric acid were added and allowed to mix for 30 minutes.

The samples were tested for photostability and the results given in TABLE 3:

**TABLE 3**

| **Material** | **Photostability** |
|---|---|
| Batch 3 | 60% |
| Batch 4 | 67% |
| Batch 5 (Comparative) | 37% |

The photostability numbers given in Table 3 are the % area under the absorbance curve that remains after 10 minutes of exposure to a UV source for a test lotion, which has been spread onto a quartz substrate with a 20 µm draw down bar. This exposure delivers a total dose of ~15 MED ("minimum erythemal dose" - the minimum radiation required to see a physiological response (redness) in a test subject) to the film. Enough of the source octinoxate in PRIMACOR dispersion or comparative material is used to have 7.5% octinoxate in the test lotion, along with an unencapsulated conventional emulsion that delivers 3% avobenzone, 4% homosalate, and 5% octylsalicylate to the test lotion.

It was observed that the inventive batches (Batch 3 and Batch 4) exhibited significantly greater photostability.

It is understood that the present invention is not limited to the embodiments specifically disclosed and exemplified herein. Various modifications of the invention will be apparent to those skilled in the art. Such changes and modifications may be made without departing from the scope of the appended claims. Moreover, each recited range includes all combinations and subcombinations of ranges, as well as specific numerals contained therein.

## Claims

1. A method for protecting low viscosity hydrophobic liquid personal care actives which have a viscosity of less than 100,000 cps (100,000 mPa.s), comprising:
forming a mixture comprising ethylene/acrylic acid copolymer, water, and a base; and
combining the active with the mixture to form suspended active particles; and
precipitating the copolymer through addition of an acid to lower the pH of the mixture, thereby forming shells around the active particles.

2. The method of claim 1, wherein the base is NaOH, KOH, or triethanolamine.

3. The method of claim 1, wherein the particle defined by the shell has a particle size, being the volume average diameter measured by light scattering with a Coulter LS230, of less than 2 µm, preferably less than 1.8 µm, preferably less than 1.6 µm, preferably less than 1.4 µm, preferably less than 1.2 µm, and preferably 1 µm.

4. The method of claim 1, wherein the step of precipitating further includes adding a stabilizer to the mixture

5. The method of claim 1, wherein the personal care active is a vitamin, emollient, sun screen, oil based pigment dispersion, essential oil, or fragrance.

6. The method of claim 1, wherein the ethylene/acrylic acid copolymer has from 9 to 22 weight percent of acrylic acid units, preferably 18 to 22 weight percent of acrylic acid units, preferably from 19 to 21 percent of acrylic acid units, and most preferably 20 acrylic acid units.

7. The method of claim 6, wherein the ratio of ethylene/acrylic acid copolymer to active in the mixture is 1:1 to 1:20, preferably 1:5 to 1:15, most preferably 1:10.

## Patentansprüche

1. Ein Verfahren zum Schützen von niedrigviskosen hydrophoben flüssigen Körperpflegewirkstoffen, die eine Viskosität von weniger als 100 000 cps (100 000 mPa.s) aufweisen, beinhaltend:
Bilden einer Mischung, die Ethylen/Acrylsäurecopolymer, Wasser und eine Base beinhaltet; und
Kombinieren des Wirkstoffs mit der Mischung, um suspendierte Wirkstoffteilchen zu bilden; und
Präzipitieren des Copolymers durch die Zugabe einer Säure, um den pH-Wert der Mischung zu senken, wodurch Hüllen um die Wirkstoffteilchen gebildet werden.

2. Verfahren gemäß Anspruch 1, wobei die Base NaOH, KOH oder Triethanolamin ist.

3. Verfahren gemäß Anspruch 1, wobei das von der Schale definierte Teilchen eine Teilchengröße, d. h. Volumendurchschnittsdurchmesser, gemessen mittels Lichtstreuung mit einem Coulter LS230, von weniger als 2 µm, vorzugsweise weniger als 1,8 µm, vorzugsweise weniger als 1,6 µm, vorzugsweise weniger als 1,4 µm, vorzugsweise weniger als 1,2 µm und vorzugsweise 1 µm aufweist.

4. Verfahren gemäß Anspruch 1, wobei der Schritt des Präzipitierens ferner das Zugeben eines Stabilisators zu der Mischung umfasst.

5. Verfahren gemäß Anspruch 1, wobei der Körperpflegewirkstoff ein Vitamin, ein Erweichungsmittel, ein Sonnenschutzmittel, eine auf ÖI basierende Pigmentdispersion, ein ätherisches Öl oder ein Duftstoff ist.

6. Verfahren gemäß Anspruch 1, wobei das Ethylen/Acrylsäurecopolymer von 9 bis 22 Gewichtsprozent Acrylsäureeinheiten, vorzugsweise 18 bis 22 Gewichtsprozent Acrylsäureeinheiten, vorzugsweise von 19 bis 21 Prozent Acrylsäureeinheiten und am besten 20 Acrylsäureeinheiten aufweist.

7. Verfahren gemäß Anspruch 6, wobei das Verhältnis des Ethylen/Acrylsäurecopolymers zu dem Wirkstoff in der Mischung 1 : 1 bis 1 : 20, vorzugsweise 1 : 5 bis 1 : 15, am besten 1 : 10 beträgt.

## Revendications

1. Une méthode pour protéger les ingrédients actifs liquides hydrophobes à faible viscosité pour soins d'hygiène personnels ayant une viscosité de moins de 100 000 cps (100 000 mPa/s), comprenant :
la formation d'un mélange comprenant un copolymère d'éthylène/acide acrylique, de l'eau, et une base ; et
la combinaison de l'ingrédient actif avec le mélange pour former des particules d'ingrédients actifs en suspension ; et
la précipitation du copolymère par le biais de l'ajout d'un acide afin d'abaisser le pH du mélange, formant de ce fait des enveloppes autour des particules d'ingrédients actifs.

2. La méthode de la revendication 1, dans laquelle la base est NaOH, KOH ou de la triéthanolanime.

3. La méthode de la revendication 1, dans laquelle la particule définie par l'enveloppe a une taille de particule, à savoir le diamètre moyen en volume mesuré par diffusion de lumière à l'aide d'un Coulter LS230, de moins de 2 µm, préférablement moins de 1,8 µm, préférablement moins de 1,6 µm, préférablement moins de 1,4 µm, préférablement moins de 1,2 µm, et préférablement 1 µm.

4. La méthode de la revendication 1, dans laquelle l'étape de précipitation inclut en sus l'ajout d'un stabilisant dans le mélange.

5. La méthode de la revendication 1, dans laquelle l'ingrédient actif pour soins d'hygiène personnels est une vitamine, un émollient, un écran solaire, une dispersion de pigments à base d'huile, une huile essentielle, ou un parfum.

6. La méthode de la revendication 1, dans laquelle le copolymère d'éthylène/acide acrylique a de 9 à 22 pour cent en poids d'unités d'acide acrylique, préférablement de 18 à 22 pour cent en poids d'unités d'acide acrylique, préférablement de 19 à 21 pour cent d'unités d'acide acrylique, et des plus préférablement 20 unités d'acide acrylique.

7. La méthode de la revendication 6, dans laquelle le rapport du copolymère d'éthylène/acide acrylique à l'ingrédient actif dans le mélange est de 1/1 à 1/20, préférablement 1/5 à 1/15, des plus préférablement 1/10.
